# EUROPEAN PATENT SPECIFICATION

(11) **EP 1 574 263 B1**
(45) Date of publication and mention of the grant of the patent: **18.02.2009**
(21) Application number: 05251347.0
(22) Date of filing: 07.03.2005
(51) Int. Cl.: B05C 3/109

(54) **Apparatus for producing a biomimetic coating on a medical implant**
Apparat zum Aufbringen einer biomimetischen Beschichtung auf ein medizinisches Implantat
Appareil pour la préparation des revêtements biomimétiques sur un implant médical

(30) Priority: 08.03.2004 US 795758
(43) Date of publication of application: 14.09.2005
(73) Proprietor: DePuy Products, Inc., Warsaw, Indiana 46580 (US)
(72) Inventor: Li, Panjian, Fort Wayne, IN 46815 (US); Hippensteel, Elizabeth, North Manchester, IN 46962 (US); Wen, Haibo, Warsaw, IN 46582 (US)
(74) Representative: Fisher, Adrian John

(56) References cited:
- WO-A-92/03232
- WO-A-99/58167
- DE-A1- 3 623 233
- DE-A1- 19 608 112
- US-A1- 2002 156 529

## Description

### FIELD OF THE INVENTION

This invention pertains to a coating for an implantable article, and more particularly, to an apparatus and method for producing a coating on an implantable article.

### BACKGROUND OF THE INVENTION

Mineralized and/or ceramic coatings are applied to a variety of articles. For example, such coatings are frequently applied to biological implants (e.g., medical implants). In the case of biological implants, the coating is usually applied to a metal or plastic substrate, but the coating can be applied to other substrates as well such as ceramic and silicon.

Biological implants, such as joint and dental prostheses, usually must be permanently affixed or anchored within bone. In some instances it is acceptable to use a bone cement to affix the prosthesis within bone. In the case of many joint prostheses, however, it is now more common to affix the joint prosthesis by encouraging natural bone ingrowth in and around the prosthesis. The bone-to-implant interfaces that result from natural bone ingrowth tend to be stronger over time and more permanent than are bone cement-prosthesis bonds.

Although various materials, including titanium alloys, are biocompatible, they are not necessarily bioactive because they can neither induce bone formation nor form chemical bonds with bone. However, providing irregular beaded or porous surfaces on the implant can enhance bone ingrowth and prosthesis fixation. For example, coating the implant with a bioactive mineralized and/or ceramic material can attain enhanced fixation of implants within bone. Such coatings have been shown to encourage more rapid bone ingrowth in and around the prosthesis. The ability of such coatings to induce bone formation can be improved by incorporating a biological agent into the bores of the ceramic coating.

Various techniques are used to apply such mineralized and/or ceramic coatings to bioimplantable substrates including using plasma spraying, ion implantation and sol-gel processing. However, each of these methods has some drawbacks. For instance, the applied coatings can be relatively thick and brittle and may not adhere well to the implant. Moreover, it can be difficult to apply the coatings on surfaces having complex geometries using these techniques resulting in uneven coating coverage and even uncoated areas. Using low temperature aqueous solutions can alleviate the problems associated with applying such coatings to implants having complex geometries. For example, an aqueous solution can be used to form a hydroapatite coating on a substrate. However, currently known low temperature processes typically require pretreatment of the substrate. These processes are also difficult to control so as to achieve a consistent uniform coating on the implant.

Accordingly, despite the existence of a variety of apparatuses and processes for producing biological agent containing coatings on implantable articles, there remains a need for an improved apparatus and process that can reliably achieve a consistent uniform coating on the implant. The invention provides such an apparatus and process. These and other advantages of the invention, as well as additional inventive features, will be apparent from the description of the invention provided herein.

An apparatus and a method according to the preamble of claims 1 and 12 is disclosed in US-A-2002/0156529.

### BRIEF SUMMARY OF THE INVENTION

The invention provides an apparatus for producing a biomimetic coating on an implantable article. The apparatus includes an outer reactor for containing a biomimetic coating solution and an inner reactor arranged within the outer reactor. The inner reactor is smaller than the outer reactor so as to define a space between the inner and outer reactors. The apparatus also includes a pump system for circulating the biomimetic coating solution. The pump system is arranged to operate in the space between the inner and outer reactors. The inner reactor includes a flow control system that is selectively operable to control the flow of biomimetic coating solution between the outer and inner reactors.

The invention further provides a method for producing a biomimetic coating on an implantable article. The method includes the step of filling an outer reactor and an inner reactor arranged within the outer reactor with a biomimetic coating solution. A flow control system associated with the inner reactor is moved to an open position so as to permit unrestrained flow of biomimetic coating solution between the outer and inner reactors. The biomimetic coating solution is mixed and heated to a desired homogeneity and temperature through a pump system that operates in a space between the outer and inner reactors. An implantable article is inserted in the inner reactor and the flow control system associated with the inner reactor is moved to a closed position so as to limit flow of biomimetic between the outer and inner reactors.

### BRIEF DESCRIPTION OF THE DRAWINGS

FIG. 1 is a perspective view of an illustrative apparatus for applying a biomimetic coating on an implantable article in accordance with the invention.

FIG. 2 is a side view of the coating apparatus of FIG. 1.

FIG. 3 is another side view of the coating apparatus of FIG. 1.

FIG. 4 is a top view of the coating apparatus of FIG. 1.

FIG. 5 is a perspective view of the coating apparatus of FIG. 1 showing some of the flow control apertures closed by stoppers.

FIG. 6 is a perspective view of an alternative embodiment of a coating apparatus according to the present invention.

FIG. 7 is a perspective view of the coating apparatus of FIG. 6 showing one of the inner reactor walls partially removed.

### DETAILED DESCRIPTION OF THE INVENTION

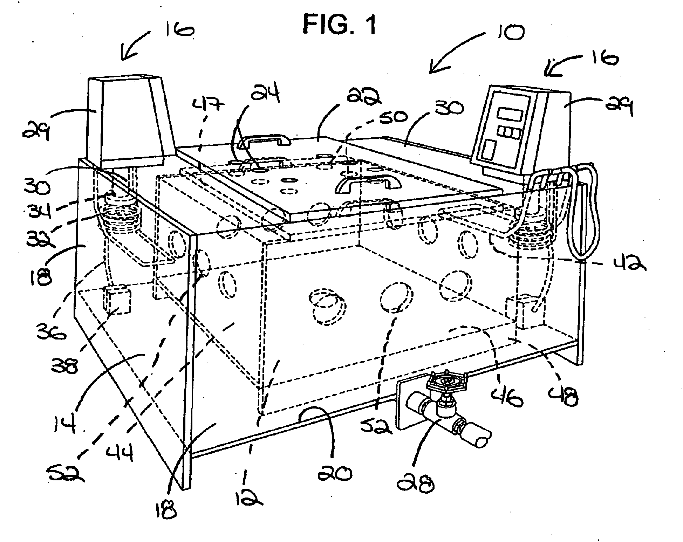

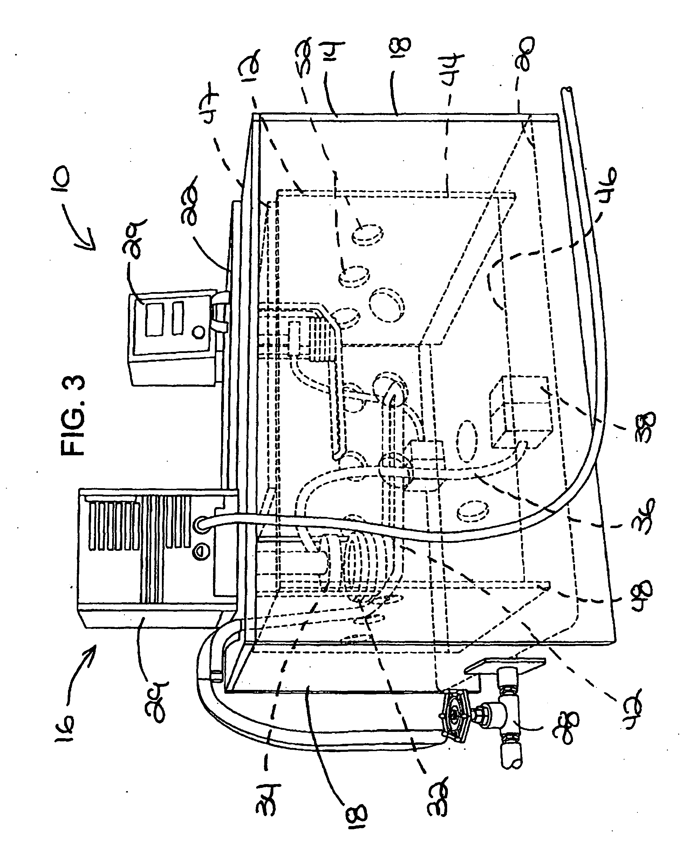

Referring more particularly to FIGS. 1-3 of the drawings, there is shown an illustrative apparatus 10 for producing a coating on a substrate, in this case an implantable article, in accordance with the present invention. More specifically, and as described in greater detail below, the illustrative apparatus 10 is capable of producing biomimetic coatings, such as for example a biomimetic apatite coating, on implantable articles. Specific details regarding such coatings and methods for preparing such coatings can be found in commonly owned U.S. Application Ser. No. 10/355,827. While the present invention is described in connection with the illustrative process of producing a biomimetic apatite coating on an implantable article, it will be readily appreciated that the invention is equally applicable to other biomimetic surface coating processes or other processes for producing bioactive surface or ceramic coatings with or without a biological agent. Moreover, the present invention could be applied in contexts such as coating processes for substrates other than implantable articles.

The composition used to form the biomimetic coating is in solution form. The solution can comprise the various chemical components suspended or dissolved in a liquid carrier. In addition, the chemical components could include a biological agent that would improve the ability of the ability of the coating to induce or conduct bone formation. For example, the composition could include a biological agent, calcium ions, phosphate ions and a liquid carrier. The liquid carrier can be any suitable aqueous or non-aqueous liquid in which the necessary chemical components for forming the bioactive surface or ceramic coating can be suspended or dissolved for delivery to the implantable article or other substrate. Some examples of suitable liquid carriers include, water, tris-buffered saline, phosphate-buffered saline, and the like.

The process for producing the biomimetic coating generally includes a solution preparation phase and a coating formation phase. In the solution preparation phase, the various chemicals that make up the particular composition that will be used to form biomimetic coating are mixed together and heated to the desired temperature. The substrates to be coated, in this case implantable articles, are then added. The implantable articles are then incubated in the solution for a period of time. The incubation time can be any suitable or desired period of time, including up to 24 hours or more or even 72 hours or more. Similarly, the incubation can take place at any suitable temperature necessary for properly preparing the biomimetic coating. For example, the incubation could take place at between about 30°C and about 50°C. Once the coating formation phase is completed, the implantable articles can be removed from the solution. Additional processing steps could include rinsing and/or drying of the implantable articles. As noted above, specific details regarding the composition of the solution and the coating process can be found in commonly owned U.S. Application Ser. No. 10/355,827.

To ensure that the coating process achieves a consistent uniform coating on the implantable articles, the illustrated apparatus 10 permits the circulation and temperature of the biomimetic coating material, such as biomimetic apatite coating material, to be tightly controlled during both the solution preparation and coating formation stages of the process. Without such tight control, the coating formed on the implantable articles can be very uneven with areas where the coating is too thin or nonexistent and areas where the coating is too thick and blocks the porous structure.

In the illustrated embodiment, the coating apparatus 10 includes inner and outer reactors 12, 14 and an associated pump system 16. The outer reactor 14 protects the integrity of the coating solution and helps maintain the temperature of the solution. In the illustrated embodiment, the outer reactor 14 has a generally rectangular configuration including two pairs of opposing sidewalls 18, a bottom wall 20 and an open top (see, e.g., FIG. 1). However, the outer reactor 14 can have any suitable or desired configuration. As can be appreciated, the outer reactor should be large enough to hold the desired amount of solution. For example, in one preferred embodiment, the outer reactor has a 110 liter capacity.

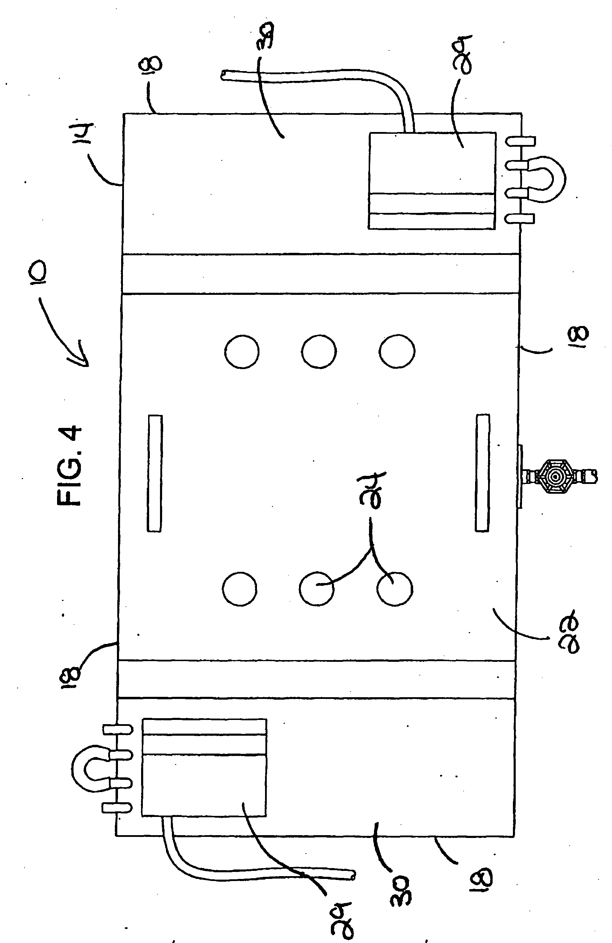

The open top of the outer reactor 14 can be large enough to allow for removal of the inner reactor 12 and/or to provide access to the inner reactor 12 for cleaning. The opening in the top of the outer reactor 14 can also be large enough to allow for manual placement of the medical implants in the inner reactor 12. The open top of the outer reactor can be covered by a removable lid 22 that sits firmly on the top of the outer reactor 14 in order to prevent excess evaporation of the solution. To accommodate external devices such as electrodes and temperature probes, the removable outer reactor lid 22 can have one or more apertures 24 therein (see, e.g., FIG. 4). The apertures 24 in the lid 22 can also be used to introduce or remove solution from the reactors 12, 14 without removing the entire lid.

The outer reactor 14 can be constructed of any suitable material including polymers or metals that can withstand the required temperatures for an extended period of time. Moreover, the material used to construct the outer reactor 14 should be strong enough to support the weight of the solution and inner reactor 12. Similarly, the lid 22 can be made of any suitable material (polymers, metals, etc.) that is capable of withstanding the required temperature for a prolonged period of time without warping.

For removal of the solution from the reactors, the outer reactor 14 can be equipped with a drainage system including a drain and a drain valve 28. The drain valve 28 can be manually operable and when opened would allow the solution to drain from the outer reactor 14 through a drain line to a sink or other receptacle for the used solution. The drain should be large enough to empty the reactors at a reasonable rate. However, the drain should be small enough to ensure that excess liquid does not collect in the tubing between the drain and the drain valve 28. All the liquid in the reactors and the drain line either from the coating process or a cleaning operation should be drained completely to prevent future coating processes from becoming contaminated.

The pump system 16 essentially controls the apparatus and the coating process by controlling the temperature and circulation rate of the biomimetic coating solution throughout the solution preparation phase and the coating formation phase. In this case, the pump system 16 is provided on the outer reactor 14. More particularly, the illustrated coating apparatus 10 includes two pump units 29 each of which is mounted on a corresponding bridge 30 that rests on the top of the outer reactor 14. These pump units 29 control the circulation of the biomimetic coating solution within the apparatus 10 so as to ensure that the chemicals are thoroughly mixed during the solution preparation phase and the homogeneity and temperature of the coating solution is maintained during the coating formation phase.

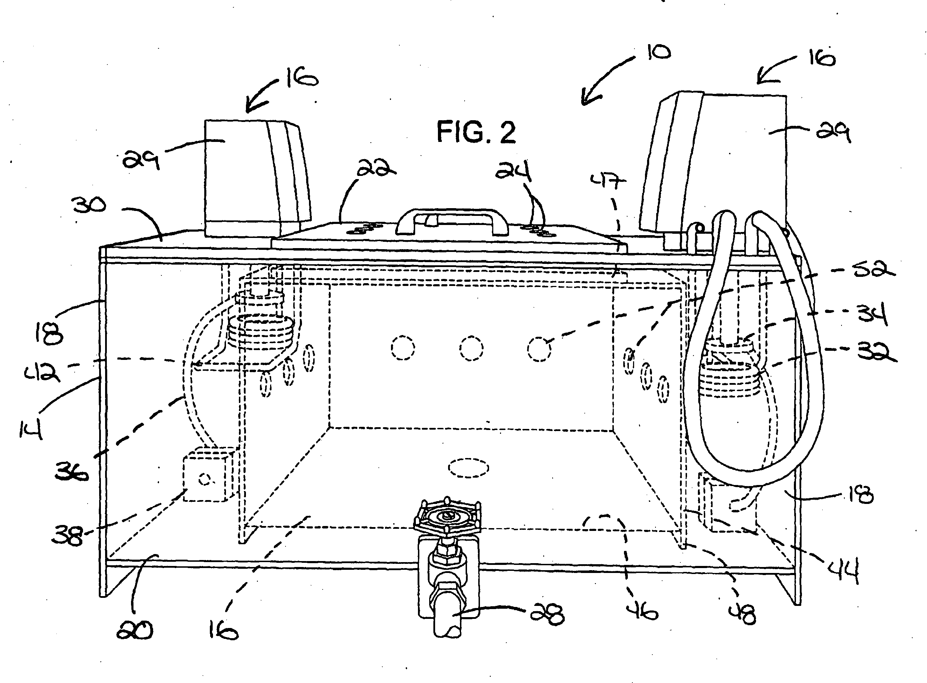

In the illustrated embodiment, each of the pump units 29 comprises an electronically controlled immersion thermostat having an integrated pump. However, it will be appreciated that separate thermostats and pumps can be provided. The illustrated pump units 29 can be setup so as to maintain the biomimetic coating solution at a set temperature and circulation rate during the solution preparation phase and again during the coating preparation phase. Each pump unit 29 has a heating coil 32 that extends downward into the outer reactor and a pump housing 34 which is also disposed inside the outer reactor 14 (see FIGS. 1 and 2). The heating coils 32 heat the biomimetic coating solution in the apparatus while the pump housings 34 provide the circulation of the solution in the apparatus 10.

In this case, a discharge tube 36 is also connected to the pump housing 34 of each of the pump units 29. These discharge tubes 36 extend downward inside the outer reactor 14 to weighted blocks 38 arranged at the bottom of the outer reactor 14 so as to direct the pump discharge along one of the sidewalls 18 of the outer reactor 14. As will be appreciated, the pump system could be setup so that the pump units discharge in any particular direction so long as sufficient circulation of the biomimetic coating solution is maintained. The illustrated pump units 29 also include outlet lines 42 that extend into the outer reactor 14 and that can be used to introduce gases into the system via corresponding inlet lines. One example of a suitable pump unit for use in the apparatus of the present invention is the Ecoline E 100 immersion thermostat with integrated pump available from Lauda of Germany. Of course, any suitable pump and thermostat could be used.

The inner reactor 12 sits within the outer reactor 14 and receives and holds the implantable articles during the coating process. For example, a positioner or platform (not shown) can be provided in the inner reactor 12 to support the implantable articles. During the coating process, the inner reactor 12 serves as a buffer to protect the implantable articles being coated from turbulence caused by the pump system 16. Specifically, the individual pump units 29 are arranged so as to operate within the space between in the inner and outer reactors 12, 14. In particular, the heating coil 32, pump housing 34, discharge tube 36 and outlet line 42 associated with each of the pump units 29 is arranged in and operates in the area between the sidewalls of the inner and outer reactors 12, 14. This arrangement helps insulate the implantable articles from turbulence or other forces caused by operation of the pump system 16.

In the illustrated embodiment, the inner reactor 12 has a generally rectangular configuration consisting of two pairs of opposing sidewalls 44, a bottom wall 46 and an open top that can be closed with a lid 47 (see FIGS. 1-3). Each of the sidewalls 44 of the inner reactor 12 extends parallel to and is shorter in length than a corresponding sidewall 18 of the outer reactor 14. The inner reactor 12 is shorter in height than the outer reactor 14 and is supported on legs 48 that space the bottom wall 46 of the inner reactor 12 above the bottom wall 20 of the outer reactor 14. As a result, space is provided between the inner and outer reactors 12, 14 on all sides of the inner reactor including above and below the inner reactor. This allows for a steady flow of the biomimetic coating solution around all sides of the inner reactor, ensuring homogeneity of the solution and a stable temperature. It will be appreciated that the inner reactor can have any desired configuration and that the space between the inner and outer reactors can also have any desired configuration.

In order to have access to the inside of the inner reactor 12 for placement of the implantable articles, the lid 47 of the inner reactor 12 can be selectively removable. The lid 47 fits firmly on the top of the inner reactor 12 so that the lid will not moved as a result of circulation of the biomimetic coating solution during the coating process. In addition, in the illustrated embodiment, the inner reactor lid 47 has a plurality of apertures 50 formed therein (see FIG. 1). These apertures 50 allow circulation of the biomimetic coating solution between the inner and outer reactors 12, 14. Moreover, these apertures 50 can be made large enough to allow external devices such as electrodes or temperature probes to have access to the solution in the inner reactor 12. The apertures 50 in the inner reactor lid 47 also provide access for removal and/or addition of biomimetic coating solution to and/or from the inner reactor 12 without disturbing the lid during the coating process.

As with the outer reactor 14, the inner reactor 12 can be constructed of any suitable material including polymers or metals that can withstand the required temperatures for an extended period of time. Moreover, the material used to construct the inner reactor should be strong enough to support the weight of the solution. Likewise, the lid 47 of the inner reactor 12 can be made of any suitable material (polymers, metals, etc.) that is capable of withstanding the required temperature for a prolonged period of time without warping.

To allow the circulation of the biomimetic coating solution within the inner reactor 12 to be controlled, the inner reactor 12 can be configured with a selectively adjustable or closable flow control system that permits the flow between the inner and outer reactors 12, 14 to be selectively adjusted. It is generally preferable that there be more circulation of the biomimetic coating solution in the inner reactor 12 in the solution preparation phase and early coating formation phase than in the later stages of the coating formation phase. Thus, the inner reactor 12 is configured so that the flow between the inner and outer reactors 12, 14 is reduced during the late stages of coating formation. This reduces the circulation of the coating solution in the inner reactor 12 thereby enhancing late stage coating formation. However, even during these later stages of the coating process, there is some circulation in the inner reactor 12 in order to maintain homogeneity of the solution and a stable temperature.

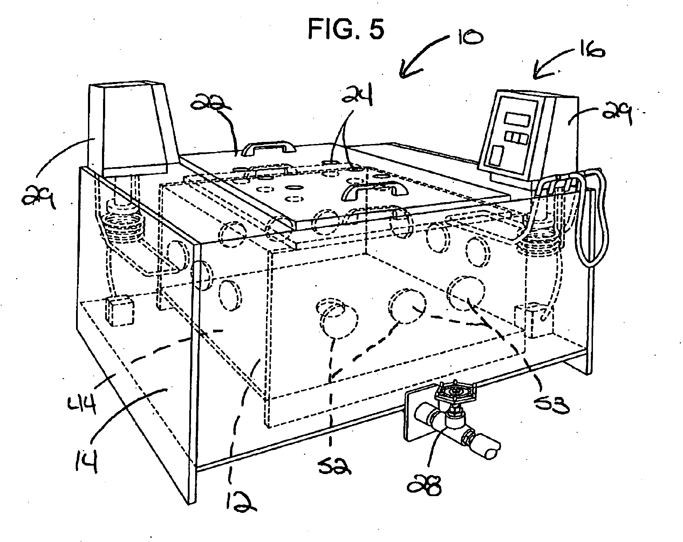

In the embodiment illustrated in FIGS. 1-5, the flow control between the inner and outer reactors 12, 14 is achieved by providing selectively closable apertures 52 in the walls of the inner reactor. In particular, one or more apertures 52 can be provided in one or more of the sidewalls 44 of the inner reactor 12. Each of these apertures 52 can be selectively opened or closed by a closure device such as a tapered stopper 53 as shown in FIG. 5. In this case, a plurality of apertures 52 are provided in each of the sidewalls 44 of the inner reactor 12 and in the bottom wall 46 of the reactor. The apertures in the bottom wall 46 of the inner rector are also used in draining the system. As noted previously, the lid 47 of the inner reactor 12 also has a plurality of apertures 50 therein. The number, size and configuration of the apertures, of course, can vary depending upon the degree of control over the flow between the reactors that is desired.

During the solution preparation phase, the majority if not all of the flow control apertures 52 in the inner reactor 12 are left open to allow for sufficient mixing of the chemicals that make up the biomimetic coating solution (see FIGS. 1-3). Later in the coating process, these flow control apertures 52 can be selectively closed so as to limit the flow between the outer and inner reactor 14, 12 and thereby reduce the circulation of the biomimetic coating solution in the inner reactor 12. The flow control apertures 52 can be closed using separate stoppers 53 that can be plugged into the apertures 52 as desired (see FIG. 5). The number of apertures 52 that are plugged closed can vary depending on the desired circulation in the inner reactor 12. Generally, at least some of the flow control apertures 52 in the inner reactor 12 are left open so as to ensure sufficient circulation to maintain the temperature and homogeneity of the solution. In the case of the illustrated embodiment, the apertures in the lid 47 and bottom wall 46 of the inner reactor 12 are typically always left open.

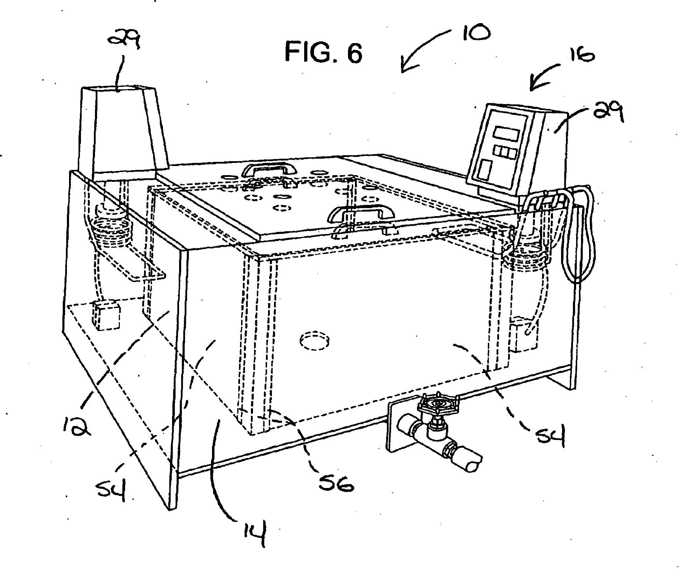

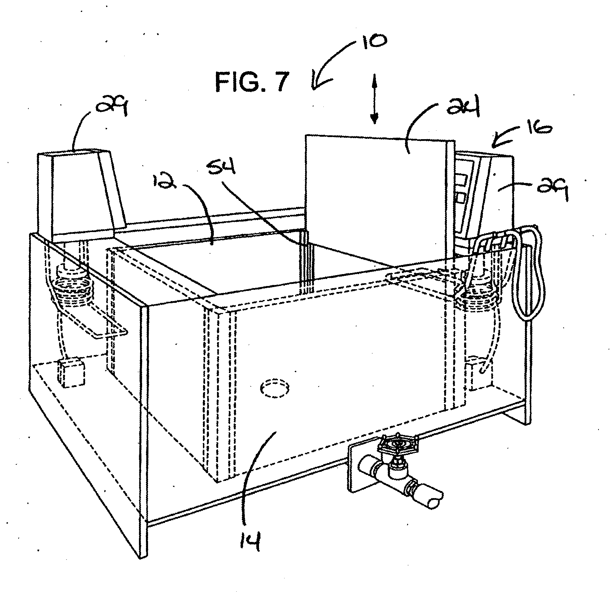

As will be appreciated the flow between the inner and outer reactors 12, 14 can be controlled in different ways and the present invention is not limited to any particular flow control system or type of flow control element. For example, an alternative embodiment of an inner reactor 12 having a different flow control system is illustrated in FIGS. 6 and 7. In this embodiment, the inner reactor 12 has a modular construction in which the sidewalls 54 can be easily removed and replaced from the reactor. In particular, the inner reactor 12 can be constructed with corner elements that define slots 56 for receiving the contiguous edges of the walls 54 defining that corner. These slots allow the walls 54 to be selectively slid into and out of place. For example, in the solution preparation phase, the walls 54 would be completely removed or slid partially up into an open position so as to maximize mixing of the solution. The solid walls 54 would then be slid into place or closed during the coating formation stage in order to limit the flow between the inner and outer reactors 12, 15. Some openings or other means would still have to be provided in the inner reactor 12, in this case openings in the lid and bottom wall, to permit sufficient circulation to maintain the temperature and homogeneity of the solution when the walls 54 were closed.

In use, to begin the solution preparation phase, the lids 22, 47 of the inner and outer reactors 12, 14 would be removed, and the chemicals making up the biomimetic coating solution would then be added to the reactors. This initial addition could be done by hand or as a part of an automated process. The pump system 16 would then be used to mix and heat the chemicals to prepare the biomimetic coating solution. During the preparation of the solution, all of the flow control apertures 52 in the inner reactor 12 would be open or, in the case of the FIGS. 6 and 7 embodiment, the walls 54 would be raised or removed. Once the solution preparation was completed, the implantable articles can be arranged in the inner reactor 12. Either at or some time after the initiation of coating formation, some or all of the flow control apertures 52 in the inner reactor 12 can be closed to limit the flow between the inner and outer reactors 12, 14. Alternatively, with the FIGS. 6 and 7 embodiment, the walls 54 could be slid into place. During coating formation, the pump system 16 would continue to operate and at least some of the flow control apertures 52 in the inner reactor 12 would remain open to maintain the temperature and homogeneity of the coating solution in the inner reactor 12. Upon completion of the coating formation phase, the implantable articles can be removed from the inner reactor 12 and subjected to any needed subsequent processing steps such as rinsing or drying. In turn, the coating solution can be drained from the inner and outer reactors, which can then be cleaned for reuse.

The term "bioactive" as used herein means having the ability to effect local tissue activity, for instance, by improving local bone formation or by preventing the onset and proliferation of microbial species.

The term "implantable article" as used herein refers to any object or device that can be inserted or embedded into or grafted onto a body, or any part thereof, and that is designed for biomedical use. The implantable article, for example, can be a bone substitute, a joint prosthesis, a dental implant (prosthodontics), a maxillofacial implant, a vertebral surgery aid, a transcutaneous device (stoma or the like), or other medical or cosmetic device. Such implantable articles can serve as a bone replacement or bone reinforcement, as well as a means of fixing a device to a particular bone.

By "biocompatible substrate" is meant any object or device that is compatible with the body into which the object or device is inserted or embedded or with the body onto which the object or device is grafted, such that the object or device will not cause an adverse immune response in the body. The biocompatible substrate can comprise any suitable material(s), such as silicon, metals, ceramics, or polymers. Biocompatible metals include titanium, tantalum, niobium, zirconium, and alloys thereof (e.g., titanium alloys and tantalum alloys), as well as cobalt-chromium alloys and stainless steel. Biocompatible polymers can be natural or synthetic polymers, such as polyethylene (e.g., ultrahigh molecular weight polyethylene or polyethylene oxide), polypropylene, polytetrafluoroethylene, polyglycolic acid, polylactic acid, other polysaccharides, and copolymers of any of the foregoing (e.g., copolymers of polylactic acid and polyglycol acid). Preferably, the biocompatible substrate comprises, consists essentially of, or consists of a biocompatible metal. More preferably, the biocompatible substrate comprises, consists essentially of, or consists of titanium. The biocompatible substrate can be any suitable portion of the implantable article, preferably a component of a prosthesis (particularly a joint prosthesis).

All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

## Claims

1. An apparatus for producing a biomimetic coating on an implantable article
**characterized by**
an outer reactor (14) for containing a biomimetic coating solution;
an inner reactor (12) for containing biomimetic coating solution arranged within the outer reactor (14), the inner reactor (12) being smaller than the outer reactor (14) so as to define a space between the inner and outer reactors;
a pump system (16) for circulating the biomimetic coating solution, the pump system (16) being arranged to operate in the pace between the inner and outer reactors (12; 14); an
wherein the inner reactor (12) includes flow control system that is selectively operable to control the flow of biomimetic coating solution between the outer and inner reactors (12; 14).

2. The apparatus according to claim 1 wherein the flow control system comprises apertures (52) in an exterior of the inner reactor (12) that are selectively closable.

3. The apparatus according to claim 2 wherein the apertures (52) are selectively closable using respective stoppers (53).

4. The apparatus according to claim 2 or 3 wherein the exterior of the inner reactor (12) includes a plurality of openings that remain open to permit circulation of the biomimetic coating solution in the inner reactor (12) when the flow control apertures are closed.

5. The apparatus according to any one of the preceding claims wherein the flow control system includes one or more walls of the inner reactor (12) that are selectively movable between open and closed positions.

6. The apparatus according to any one of the preceding claims wherein the space between the outer and inner reactors (12; 14) extends around substantially the entire exterior of the inner reactor (12).

7. The apparatus according to any one of the preceding claims wherein the pump system (16) is supported on the outer reactor (14) and discharges in the space between the inner and outer reactors (12; 14).

8. The apparatus according to any one of the preceding claims wherein the pump system (16) heats the biomimetic coating solution.

9. The apparatus according to any one of the preceding claims wherein the inner and/or outer reactor (12; 14) has an opening in a top thereof that is selectively closable by a lid (22; 47).

10. The apparatus according to claim 9 wherein the or each reactor lid (22; 47) has apertures therein.

11. The apparatus according to any one of the preceding claims wherein both the inner and outer reactors (12; 14) have a generally rectangular configuration.

12. A method for producing a biomimetic coating on an implantable article **characterized by** the steps of, in any particular order:
filling an outer reactor (14) and an inner reactor (12) arranged within the outer reactor (14) with a biomimetic coating solution;
moving a flow control system associated with the inner reactor (12) to an open position so as to permit unrestrained flow of biomimetic coating solution between the outer (14) and inner (12) reactors;
mixing and heating the biomimetic coating solution to a desired homogeneity and temperature through a pump system (16) that operates in a space between the outer and inner reactors (12; 14);
inserting an implantable article in the inner reactor (12); and
moving the flow control system associated with the inner reactor (12) to a closed position so as to limit flow of biomimetic between the outer and inner reactors (12; 14).

13. The method according to claim 12 wherein the flow control system comprises apertures (52) in an exterior of the inner reactor (12) that are selectively closable.

14. The method according to claim 13 wherein the flow control apertures (52) are selectively closable using respective stoppers (53).

15. The method according to claim 12 or 13 wherein the flow control system comprises one or more walls of the inner reactor (12) that are selectively movable between open and closed positions.

16. The method according to any one of claims 12 to 15 wherein the space between the inner and outer reactors (12; 14) extends substantially around substantially the entire exterior of the inner reactor (12).

17. The method according to any one of claims 12 to 16 wherein biomimetic solution circulates in the inner reactor (12) when the inner reactor flow control system is closed as a result of openings in the inner reactor (12).

18. The method according to any one of claims 12 to 17 wherein the step of inserting an implantable article in the inner reactor (12) includes removing an inner reactor lid (47) covering an opening in the inner reactor (12).

19. The method according to claim 18 further including the step of inserting an external device into the biomimetic solution through an aperture (50) in the inner reactor lid (47).

20. The method according to any one of claims 12 to 19 wherein the step of inserting an implantable article in the inner reactor (12) include removing an outer reactor lid (22) covering an opening in the outer reactor (14).

21. The method according to claim 20 further including the step of inserting an external device into the biomimetic solution through an aperture in the outer reactor lid (22).

## Patentansprüche

1. Vorrichtung zum Erzeugen einer biomimetischen Beschichtung auf einem implantierbaren Objekt, **gekennzeichnet durch**
einen äußeren Reaktor (14) zum Aufnehmen einer biomimetischen Beschichtungslösung;
einen innen Reaktor (12) zum Aufnehmen einer biomimetischen Beschichtungslösung, der innerhalb des äußeren Reaktors (14) angeordnet ist, wobei der innere Reaktor (12) kleiner als der äußere Reaktor (14) ist, um so einen Raum zwischen dem inneren und dem äußeren Reaktor zu definieren;
ein Pumpensystem (16) zum Umwälzen der biomimetischen Beschichtungslösung, wobei das Pumpensystem (16) so angeordnet ist, dass es in dem Raum zwischen dem inneren und dem äußeren Reaktor (12; 14) arbeitet; und
wobei der innere Reaktor (12) ein Strömungssteuersystem umfasst, das ausgewählt betreibbar ist, um den Strom der biomimetischen Beschichtungslösung zwischen dem äußeren und dem inneren Reaktor (12; 14) zu steuern.

2. Vorrichtung nach Anspruch 1, bei der das Strömungssteuersystem Öffnungen (52) in einem Außenbereich des inneren Reaktors (12) aufweist, die ausgewählt verschließbar sind.

3. Vorrichtung nach Anspruch 2, bei der die Öffnungen (52) ausgewählt verschließbar sind, indem jeweilige Sperrelemente (53) verwendet werden.

4. Vorrichtung nach Anspruch 2 oder 3, bei der der Außenbereich des inneren Reaktors (12) eine Vielzahl von Öffnungen umfasst, die offen bleiben, um das Umwälzen der biomimetischen Beschichtungslösung in dem inneren Reaktor (12) zu erlauben, wenn die Öffnungen zur Strömungssteuerung geschlossen sind.

5. Vorrichtung nach einem der vorangehenden Ansprüche, bei der das Strömungssteuersystem eine oder mehrere Wände des inneren Reaktors (12) umfasst, die wahlweise zwischen einer Offen- und einer Schließstellung bewegbar sind.

6. Vorrichtung nach einem der vorangehenden Ansprüche, bei der der Raum zwischen dem äußeren und dem inneren Reaktor (12; 14) sich um im Wesentlichen den gesamten Außenbereich des inneren Reaktors (12) erstreckt.

7. Vorrichtung nach einem der vorangehenden Ansprüche, bei der das Pumpensystem (16) auf dem äußeren Reaktor (14) gehalten ist und in den Raum zwischen dem inneren und dem äußeren Reaktor (12; 14) abfördert.

8. Vorrichtung nach einem der vorangehenden Ansprüche, bei der das Pumpensystem (16) die biomimetische Beschichtungslösung heizt.

9. Vorrichtung nach einem der vorangehenden Ansprüche, bei der der innere und/oder der äußere Reaktor (12; 14) eine Öffnung in seiner Oberseite hat, die wahlweise durch einen Deckel (22; 47) verschließbar ist.

10. Vorrichtung nach Anspruch 9, bei der der oder jeder Reaktordeckel (22; 47) Öffnungen enthält.

11. Vorrichtung nach einem der vorangehenden Ansprüche, bei der sowohl der innere als auch der äußere Reaktor (12; 14) eine im Allgemeine rechtwinklige Ausgestaltung hat.

12. Verfahren zum Erzeugen einer biomimetischen Beschichtung auf einem implantierbaren Objekt, **gekennzeichnet durch** die Schritte, in jedweder bestimmten Reihenfolge;
Befüllen eines äußeren Reaktors (14) und eines inneren Reaktors (12), der innerhalb des äußeren Reaktors (14) angeordnet ist, mit einer biomimetischen Beschichtungslösung;
Bewegen eines Strömungssteuersystems, das dem inneren Reaktor (12) zugeordnet ist, in eine Offenstellung, um somit einen unbeschränkten Strom der biomimetischen Beschichtungslösung zwischen dem äußeren (14) und dem inneren (12) Reaktor zu erlauben;
Mischen und Heizen der biomimetischen Beschichtungslösung auf eine gewünschte Homogenität und Temperatur **durch** ein Pumpensystem (16), das in einem Raum zwischen dem äußeren und dem inneren Reaktor (12; 14) arbeitet;
Einsetzen eines implantierbaren Objektes in den inneren Reaktor (12); und
Bewegen des Strömungssteuersystems, das dem inneren Reaktor (12) zugeordnet ist, in eine Schließstellung, um so den Strom der biomimetischen Lösung zwischen dem äußeren und dem inneren Reaktor (12; 14) zu begrenzen.

13. Verfahren nach Anspruch 12, bei dem das Strömungssteuersystem Öffnungen (52) in einem Außenbereich des inneren Reaktors (12) aufweist, die ausgewählt verschließbar sind.

14. Verfahren nach Anspruch 13, bei dem die Strömungssteueröffnungen (52) ausgewählt verschließbar sind, indem jeweilige Sperrelemente (53) verwendet werden.

15. Verfahren nach Anspruch 12 oder 13, bei dem das Strömungssteuersystem eine oder mehrere Wände des inneren Reaktors (12) aufweist, die ausgewählt zwischen einer Offen- und einer Schließstellung bewegbar sind.

16. Verfahren nach einem der Ansprüche 12 bis 15, bei dem sich der Raum zwischen dem inneren und dem äußeren Reaktor (12; 14) im Wesentlichen um im Wesentlichen den gesamten Außenbereich des inneren Reaktors (12) erstreckt.

17. Verfahren nach einem der Ansprüche 12 bis 16, bei dem biomimetische Lösung in dem inneren Reaktor (12) umläuft, wenn das Strömungssteuersystem des inneren Reaktors aufgrund der Öffnungen in dem inneren Reaktor (12) geschlossen ist.

18. Verfahren nach einem der Ansprüche 12 bis 17, bei dem der Schritt des Einsetzens eines implantierbaren Objektes in den inneren Reaktor (12) das Entfernen eines Deckels (47) des inneren Reaktors umfasst, der eine Öffnung in dem inneren Reaktor (12) abdeckt.

19. Verfahren nach Anspruch 18, das weiter den Schritt des Einsetzens einer externen Einheit in die biomimetische Lösung durch eine Öffnung (50) in dem Deckel (47) des inneren Reaktors umfasst.

20. Verfahren nach einem der Ansprüche 12 bis 19, bei dem der Schritt des Einsetzens eines implantierbaren Objektes in den inneren Reaktor (12) das Entfernen eines Deckels (22) des äußeren Reaktors, der eine Öffnung in dem äußeren Reaktor (14) abdeckt, umfasst.

21. Verfahren nach Anspruch 20, das weiter den Schritt des Einsetzens einer externen Einheit in die biomimetische Lösung durch eine Öffnung in dem Deckel (22) des äußeren Reaktors umfasst.

## Revendications

1. Appareil pour produire un revêtement biomimétique sur un élément implantable, **caractérisé par**
■ un réacteur externe (14) destiné à contenir une solution de revêtement biomimétique ;
■ un réacteur interne (12) destiné à contenir une solution de revêtement biomimétique agencé dans le réacteur externe (14), le réacteur interne (12) étant plus petit que le réacteur externe (14) de manière à définir un espace entre les réacteurs interne et externe ;
■ un système de pompe (16) pour faire circuler la solution de revêtement biomimétique, le système de pompe (16) étant agencé pour fonctionner dans l'espace entre les réacteurs interne et externe (12 ; 14) ; et
■ dans lequel le réacteur interne (12) comprend un système de commande d'écoulement qui peut être mis en oeuvre de manière sélective pour commander l'écoulement de la solution de revêtement biomimétique entre les réacteurs interne et externe (12 ; 14).

2. Appareil selon la revendication 1, dans lequel le système de commande d'écoulement comprend des ouvertures (52) dans une partie extérieure du réacteur interne (12) qui peuvent être fermées de manière sélective.

3. Appareil selon la revendication 2, dans lequel les ouvertures (52) peuvent être fermées de manière sélective en utilisant des butées (53) respectives.

4. Appareil selon la revendication 2 ou 3, dans lequel l'extérieur du réacteur interne (12) comprend une pluralité d'ouvertures qui restent ouvertes pour permettre la circulation de la solution de revêtement biomimétique dans le réacteur interne (12) lorsque les ouvertures de commande d'écoulement sont fermées.

5. Appareil selon l'une quelconque des revendications précédentes, dans lequel le système de commande d'écoulement comprend une ou plusieurs parois du réacteur interne (12) qui peuvent être déplacées de manière sélective entre des positions ouverte et fermée.

6. Appareil selon l'une quelconque des revendications précédentes, dans lequel l'espace entre les réacteurs interne et externe (12 ; 14) s'étend autour de l'extérieur sensiblement entier du réacteur interne (12).

7. Appareil selon l'une quelconque des revendications précédentes, dans lequel le système de pompe (16) est supporté sur le réacteur externe (14) et refoule dans l'espace entre les réacteurs interne et externe (12 ; 14).

8. Appareil selon l'une quelconque des revendications précédentes, dans lequel le système de pompe (16) chauffe la solution de revêtement biomimétique.

9. Appareil selon l'une quelconque des revendications précédentes, dans lequel les réacteurs interne et/ou externe (12 ; 14) comportent une ouverture dans une partie supérieure de ceux-ci qui peut être fermée de manière sélective par un couvercle (22 ; 47).

10. Appareil selon la revendication 9, dans lequel le ou chaque couvercle de réacteur (22 ; 47) comporte des ouvertures dans celui-ci.

11. Appareil selon l'une quelconque des revendications précédentes, dans lequel les réacteurs interne et externe (12 ; 14) ont tous deux une configuration généralement rectangulaire.

12. Procédé pour produire un revêtement biomimétique sur un élément implantable, **caractérisé par** les étapes consistant à, dans n'importe quel ordre particulier :
■ remplir un réacteur externe (14) et un réacteur interne (12) agencé dans le réacteur externe (14) d'une solution de revêtement biomimétique ;
■ déplacer un système de commande d'écoulement associé au réacteur interne (12) dans une position ouverte de manière à permettre un écoulement non limité de solution de revêtement biomimétique entre les réacteurs externe (14) et interne (12) ;
■ mélanger et chauffer la solution de revêtement biomimétique à une homogénéité et une température souhaitées par l'intermédiaire d'un système de pompe (16) qui fonctionne dans un espace entre les réacteurs interne et externe (12 ; 14) ;
■ insérer un élément implantable dans le réacteur interne (12) ; et
■ déplacer le système de commande d'écoulement associé au réacteur interne (12) dans une position fermée de manière à limiter l'écoulement de revêtement biomimétique entre les réacteurs interne et externe (12 ; 14).

13. Procédé selon la revendication 12, dans lequel le système de commande d'écoulement comprend des ouvertures (52) dans une partie extérieure du réacteur interne (12) qui peuvent être fermées de manière sélective.

14. Procédé selon la revendication 13, dans lequel les ouvertures de commande d'écoulement (52) peuvent être fermées de manière sélective en utilisant des butées (53) respectives.

15. Procédé selon la revendication 12 ou 13, dans lequel le système de commande d'écoulement comprend une ou plusieurs parois du réacteur interne (12) qui peuvent être déplacées de manière sélective entre des positions ouverte et fermée.

16. Procédé selon l'une quelconque des revendications 12 à 15, dans lequel l'espace entre les réacteurs interne et externe (12 ; 14) s'étend sensiblement autour de l'extérieur sensiblement entier du réacteur interne (12).

17. Procédé selon l'une quelconque des revendications 12 à 16, dans lequel une solution biomimétique circule dans le réacteur interne (12) lorsque le système de commande d'écoulement de réacteur interne est fermé du fait des ouvertures dans le réacteur interne (12).

18. Procédé selon l'une quelconque des revendications 12 à 17, dans lequel l'étape d'insertion d'un élément implantable dans le réacteur interne (12) comprend le retrait d'un couvercle de réacteur interne (47) recouvrant une ouverture dans le réacteur interne (12).

19. Procédé selon la revendication 18, comprenant en outre l'étape consistant à insérer un dispositif externe dans la solution biomimétique à travers une ouverture (50) dans le couvercle de réacteur interne (47).

20. Procédé selon l'une quelconque des revendications 12 à 19, dans lequel l'étape d'insertion d'un élément implantable dans le réacteur interne (12) comprend le retrait d'un couvercle de réacteur externe (22) recouvrant une ouverture dans le réacteur externe (14).

21. Procédé selon la revendication 20, comprenant en outre l'étape d'insertion d'un dispositif externe dans la solution biomimétique à travers une ouverture dans le couvercle de réacteur externe (22).
